(19) Europäisches Patentamt
European Patent Office
Office européen des brevets

(11) Publication number: **0 238 225 B1**

(12) # EUROPEAN PATENT SPECIFICATION

(45) Date of publication of patent specification: **15.07.92** (51) Int. Cl.$^5$: **A61K 7/021**

(21) Application number: **87301682.8**

(22) Date of filing: **26.02.87**

(54) **Cosmetic colorant compositions.**

(30) Priority: **18.03.86 US 840917**

(43) Date of publication of application:
**23.09.87 Bulletin 87/39**

(45) Publication of the grant of the patent:
**15.07.92 Bulletin 92/29**

(84) Designated Contracting States:
**DE FR GB IT**

(56) References cited:
**FR-A- 2 033 286** **GB-A- 1 298 263**
**JP-A- 6 049 319** **LU-A- 57 903**
**US-A- 3 609 102** **US-A- 4 309 411**

(73) Proprietor: **MINNESOTA MINING AND MANUFACTURING COMPANY**
**3M Center, P.O. Box 33427**
**St. Paul, Minnesota 55133-3427(US)**

(72) Inventor: **Sweeny, Norman P. c/o Minnesota Mining and**
**Manufacturing Company 2501 Hudson Road**
**P.O. Box 33427 St. Paul Minn. 55133-3427(US)**

(74) Representative: **Baillie, Iain Cameron et al**
**c/o Ladas & Parry, Altheimer Eck 2**
**W-8000 München 2(DE)**

**EP 0 238 225 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to compositions useful in the application of colored cosmetics to the human body.

The application of decorative or masking colors to the skin of humans and particularly to the face of humans is a highly specialized technical area. Compositions vary greatly depending upon the specific location to which the color is to be applied. The compositions used to apply color to the lips (e.g., lipstick or lip balm) require water repellancy because of their repeated contact with liquids. Mascara must have very high color density and strong bonding ability to eyelashes. Rouge, blusher and pancake must be very fine and easily spreadable. Eye shadow must also be fine and spreadable, yet should not break down during mild abrasion as is encountered with repeated blinking and movement of eyelids. There are also bases and foundations for colors which require specific properties to enable them to perform their unique function.

In recent years, styles have become more dramatic, allowing the use of more color and even multiple colors in the applications of makeup. For example, one portion of an eyelid may be pink and another portion blue. The change in colors can be used not only for the multicolor appearance, but the change can give an appearance of eyes being farther apart or closer together then they actually are. Such make-over techniques are highly valued by the cosmetician and makeup artist.

There are some problems involved in blending colors, however. Even though separate color sources and separate brushes may be used to apply the individual colors, the later applied colors will always contact the first applied colors. The brushes will then pick up colorant from the skin and transport it back to the colour source. The brushes themselves will also retain mixtures of colors, rendering them less useful. The intermixing of colors in the original source will of course diminish the quality of the colorant.

It is fairly common to find encapsulated liquid materials in the marketplace. Technology has been available for many years to effectively provide microcapsules with liquid oleophilic ingredients. Representative processes are shown in U.S. Patents 3,0l6,308 and 3,5l6,94l. These patents disclose in situ polymerization reactions in which a hydrophobic oil phase is dispersed in an aqueous phase containing resin precursors, particularly aminoplast resin precursors (to form urea/aldehyde resins and the like). High shear agitation is used to keep the capsule size small. Addition of an acid catalyst initiates the polycondensation of the aminoplast precursors, resulting in the deposition of the aminoplast resin about the dispersed droplets of the oil phase. This produces the microcapsules.

Other polycondensations encapsulation techniques are shown in U.S. Patents 3,429,827 and 4,000,087. These particular techniques are more limited in the classes of hydrophobic inner phases acceptable in the microcapsules because of reaction with the oil soluble monomer or poor solubility of the monomer in the desired hydrophobic phase.

U.S. Patent 3,930,l0l teaches that, to be retained in the hydrophobic phase during high shear dispersion of a fluid particulate dispersion, it is necessary that the particculate be preferentially wetted by the hyrophobic phase. It is suggested to use suitable surfactants which adsorb to the particulate surface as a way to achieve the desired preferential wetting. It has, however, been recognized that, in the in situ polymerization of aminoplast resins method for encapsulaton, the presence of surfactants interferes with the deposition of the aminoplast resin at the hydrophobic phase/water phase interface, giving poorly formed or leaky capsules. Similarly, oil soluble suspending agents could alter the wetting of many particulates. Since many of these materials contain carboxylate groups, exposure to highly acidic medias often converts them to carboxylic acid groups altering their adsorbability to the particulates.

U.S. Patent 4,307,169 teaches the inclusion of magnetic materials into a pressure fixable core material within a shell formed by interfacial polycondensation.

U.S. Patent 3,954,666 teaches the preparation of semipermeable microcapsules containing catalysts and ferromagnetic materials.

U.S. Patent 4,450,221 teaches magnetic toners comprising lyophilic magnetic particles and a resin surrounded by a resin wall to form microcapsules. Colorants such as pigments or dyes may be included in the wall forming resin or the toner. The magnetic particles are rendered lyophilic by treatment with a titanate or silane coupling agent. The coupling agent is said to uniformly disperse the particles in the binder resin and firmly bond the magnetic particle to the resin.

GB-A-1 298 263 discloses pressurized microcapsules containing an ingredient which has to be stored separately from another incompatible ingredient. Both ingredients are bought into contact when the pressurized microcapsules burst on exposure to atmospheric pressure.

FR-A-2 033 286 describes a dye composition in which part of the colorants are encapsulated. The microcapsules are pressurized and they explode under atmospheric pressure.

Conventional cosmetic formulations, with or without a predominant coloration in the composition, can be

rendered colored, can be highlighted, or can be altered in color by the application of pressure to the composition after or during application. The presence within the composition of frangible microcapsules containing pigment enables this coloring phenomenon to occur.

Color adjustable cosmetic compositions comprise a first colorant, a binder, and frangible microcapsules comprising a shell, an encapsulated liquid and a second colorant which differs in color from said first colorant, wherein the cosmetic composition undergoes a visible color change only upon rupture of the microcapsules, said microcapsules being rupturable by rubbing of the composition after it has been applied to skin.

In other embodiments compositions are disclosed where the composition being a compact face powder comprises 2-25% by weight binder, 20-75% by weight first colorant and 10-78% by weight microcapsules; where the composition being a cream rouge comprises 35-65% cream base, 15-50% first colorant and 15-50% microcapsules; where the composition being a compact eye shadow comprises 30-70% talc, 2-12% metal carboxylate, 2-10% of a binder selected from waxes and lipids, 4-30% first colorant and 3-30% microcapsules and optionally further comprises up to 12% by weight of a white pigment which can be titanium dioxide; and where the composition being a lipstick comprises 30-70% by weight of a binder selected from lipids and waxes, 15-40% by weight of a solvent for said binder, 3-15% by weight of first colorant and 3-15% by weight of microcapsules.

The present invention relates to cosmetic formulations, with or without a predominant coloration in the compositions, which by the application of differential pressure over the surface of the composition, during or after application, can be rendered colored, can be highlighted, or can be altered in its predominant color. Conventional cosmetic color compositions such as eye shadow, lipstick, pancake, face powder, lip balm, cream rouge, mask powder, suntan lotion, and the like are used as the basic compositions of the invention. To these basic compositions are added microcapsules containing pigments of colors (including sparkling pigments) which may be different from the color in the original composition. After application of the composition, further light rubbing (as with a finger) will rupture the microcapsules, highlighting or changing the color in the area where the composition was rubbed by exposing the encapsulated pigment within the composition.

In accordance with the present invention, microcapsules are prepared by in situ such as aminoplast polymerization. The techniques disclosed, generally referred to as an in situ polymerization reaction, yield, for example, an aminoplast resin capsule wall material. In the process, a hydrophobic oil phase is dispersed in an aqueous phase containing the aminoplast resin precursors by applying high shear agitation. Addition of an acid catalyst initiates the polycondensation of the aminoplast precursors, resulting in the deposition of the aminoplast resin about the dispersed droplets of the oil phase, producing the microcapsules.

Pigments of various types, when dispersed in water insoluble oils, and then mixed or dispersed under high shear into water phases to produce oil in water dispersions, will show a variety of behaviors depending upon the surface characteristics of the pigment particles relative to the oil and water phases. In particular, pigment particles which are wetted by the water phase and incompletely or poorly wetted by the oil phase, will readily move from the oil phase to the water phase during this dispersion process. Attempts to encapsulate such a pigment will generally be unsuccessful and lead to a capsule containing few, if any, pigment particles. Pigment particles which are completely wetted by the water phase will tend to remain in the interior of the oil phase droplets during the dispersion process. Pigment particles of this type will generally result in microcapsules having the pigment particles in the oil core of the microcapsule with relatively few particles being abstracted from the oil phase or caught or immobilized in the microcapsule shell wall. Finally, pigment particles which are incompletely wetted by either the oil phase or by the water phase will be found concentrated at the oil/water interface during such a dispersion process. Microcapsules formed from this type of pigment will give capsules having the pigment particles more or less in the shell wall of the microcapsule. The ability to alter the surface characteristics and more particularly the wetting characteristics of pigment particles relative to the chosen oil and water phase compositions provides the means to control the encapsulatability of pigment dispersions and the means to control the ultimate location of the majority of the pigment particles within the microcapsule (i.e., either freely dispersed in the core oil phase or fixed at or within the microcapsule shell wall). Useful products can be made from microcapsules containing pigments of either the two types.

It is part of this invention that the second colorant be associated with the microcapsules. This means that the second colorant should be on the shell, in the shell, or within the shell of the microcapsule. The more of the second pigment that is within the shell, the greater will be the degree change in the overall coloration of the product upon rupture of the shells. That is because any second pigment (colorant) on the exterior of the shell will contribute to the overall color of the composition before rupture of the microcapsules. It is therefore preferred that said second colorant is predominantly encapsulated by the shell. This

means that at least 50% by weight of said second colorant is within the shell. Amounts as small as 25% by weight of said second colorant within the capsules (in the shell wall and inside the shell itself) can significantly alter the color or tone of the composition on being ruptured. It is preferred that at least 50% by weight of all second colorant be inside the shell (on the wall, or in the liquid carrying medium, or in the wall). More preferably at least 70% is inside the shell, and most preferably more than 90% of said second colorant is inside the shell.

The preferred method of forming capsules for use in the present invention is a process whereby the surface of non-magnetic colorant particulates, by the addition of surface adsorbable agents, are rendered oleophilic and can maintain their oleophilicity in water (at pH's encountered in encapsulation processes). This may mean pH's of 10 or more for some interfacial encapsulation processes or pH 4-1.8 for in-situ aminoplast encapsulation. The additive must remain on the surface of the particle for a period of at least five minutes at a pH representative of the extreme pH to be encountered in the encapsulation process under the following conditions:

1. The oil phase used as the interior phase of the capsule (e.g., diethyl phthalate) is placed in the same flask with the pigment. The pigment is either pretreated with the additive or the pigment is treated in-situ by incorporation of additive into the oil phase.
2. One part of the oil phase dispersion to ten parts of an aqueous phase at the required pH are combined in a flask.
3. The combination in the flask is shaken vigorously for at least five (and preferably ten) minutes. The phases are microscopically examined to determine the location of the pigment. If at least 20%, preferably no more than at least 10%, and no more than at least 5% of the pigment is in the water phase (unassociated with oil phase), the additive has failed.

This test procedure defines the functional ability of an oleophilic additive according to the present invention. Any material that passes this test (no more than 20% in the water phase) is referred to as a functional oleophilic additive.

It has been found that two main classes of pigment surface modifying agents are particularly useful for controlling the wetting characteristics of a variety of pigment types. These materials are usually described as titanate or silan coupling agents. Judicious selection of the coupling agents from these classes allows the control of the encapsulatability and particulate location of a variety of pigment types, associated with a variety of different oil phase compositions. Three methods for the use of these surface modifying agents are possible depending upon the specific agent chosen and on other restrictions dictated by the microcapsule use. They are:

1. Pretreatment of the pigment particles prior to dispersion in the oil phase.
2. Addition of the coupling agent directly to the oil phase of the pigment dispersion.
3. Having the coupling agent present in the water phase at the time of dispersing of the oil/pigment dispersion.

The first process is the most universally useful as the pretreatments are usually accomplished by exposure of the pigments to solutions of the coupling agents. Selection of solvents according to the nature of the coupling agent is more readily accomplished. For example, the preferred solvent, and sometimes the required solvent, for use with many of the silane coupling agents is water. In other cases, the solubility of a chosen coupling agent in the desired oil phase for encapsulation may not be satisfactory for effective pigment treatment, or it may be undesirable to have free coupling agent present in the oil phase of the encapsulated pigment dispersion. Treatment of the pigment in solutions of 0.05% to 10% of the coupling agent will usually produce acceptable results, with 0.25% to 2.0% being most desirable.

The second method is restricted to coupling agents of the two classes that have solubility in the oil phase composition to be used in the microcapsule. Usually an excess of the coupling agent over that necessary for the surface treatment of the pigment will be used. This will result in some residual soluble coupling agent present in the oil/pigment dispersion. The addition of .05% to 5% by weight of the particulate will usually produce acceptable results.

The third method is restricted to those coupling agents which are soluble in water. It is most useful with pigments which are wettable by water. Additions of 0.05% to 10% of the coupling agent by weight of the particulate will usually produce acceptable results, with 0.25% to 2.0% being the most desired range.

Pigments as used in the present invention refer exclusively to solid materials. Dyes or pigments carried in solid polymeric or waxy phases can constitute pigments in the present invention, but dyes dissolved in liquid media are not solids and therefore not within the definition of pigments.

Chemisorption of the additives to the particulates is preferred, but not essential. Adsorption of the additives to the particulates (e.g., pigments) by Van der Waals forces, dipole-dipole attraction, or hydrogen bonding are also useful. Chemisorption requires an actual chemical bond to be formed between a part of

the additive and the particulate surface. The pigments for which this process is most useful are those which have hydrophilic surfaces initially and require increased oleophilicity of their surfaces. The pigments may be a single, solid particulate, colorant materials carried in a solid medium or colloidal materials which in gross or bulk form appear gelatinous (e.g., colloidal, hydrated iron oxide) and thus act as a solid.

Titanate coupling agents that are illustrative of those used in the present invention as agents to modify the wettability of the particulates have formulas shown in Table I.

TABLE I

| | |
|---|---|
| 1. | Isopropyl triisostearoyl titanate |
| 2. | Isopropyl methacryl diisostearoyl titanate |
| 3. | Isopropyl dimethacryl isostearoyl titanate |
| 4. | Isopropyl tridodecylbenzenesulfonyl titanate |
| 5. | Isopropyl diacryl isostearoyl titanate |
| 6. | Isopropyl tri(dioctylphosphato) titanate |
| 7. | Isopropyl 4-aminobenzenesulfonyl di(dodecylbenzenesulfonyl titanate |
| 8. | Isopropyl trimethacryl titanate |
| 9. | Isopropyl tricumylphenyl titanate |
| 10. | Isopropyl di(4-aminobenzoyl) isostearoyl titanate |
| 11. | Isopropyl tri(dioctylpyrophosphato) titanate |
| 12. | Isopropyl triacryl titanate |
| 13. | Isopropyl tri(N ethylamino-ethylamino) titanate |
| 14. | Isopropyl tri(2-aminobenzoyl) titanate |
| 15. | Isopropyl tri(butyl, octyl pyrophosphato) titanate di(dioctyl, hydrogen) phosphate |
| 16. | Di(butyl, methyl pyrophosphato) isopropyl titanate di(dioctyl, hydrogen) phosphite |
| 17. | Titanium isostearate methacrylate oxyacetate |
| 18. | Titanium acrylate isostearate oxyacetate |
| 19. | Titanium dimethacrylate oxyacetate |
| 21. | Titanium di(cumylphenylate) oxyacetate |
| 22. | Titanium di(dioctylpyrophosphate) oxyacetate |
| 23. | Titanium diacrylate oxyacetate |
| 24. | Titanium di(butyl, octyl pyrophosphate) di(dioctyl, hydrogen phosphite) oxyacetate |
| 25. | Diisostearoyl ethylene titanate |
| 26. | Di(dioctylphosphato) ethylene titanate |
| 27. | 4-aminobenzenesulfonyl dodecylbenzenesulfonyl ethylene titanate |
| 28. | Di(dioctylpyrophosphato) ethylene titanate |
| 29. | Di(butyl, methyl pyrophosphato)ethylene titanate di(dioctyl, hydrogen phosphite) |
| 30. | Tetraisopropyl di(dioctylphosphito) titanate |
| 31. | Tetraoctyloxytitanium di(ditridecylphosphite) |
| 32. | Tetra(2,diallyoxymethyl-1 butoxy titanium di(di-tridecyl)phosphite |

The above listed titanate coupling agents are commercially available.

Illustrative silane coupling agents have the formulas below:

$RSiX_3$
$RR'SiX_2$
$RR'R''SiX$

Where X is Cl, alkoxy of 1-4 carbon atoms (e.g. $OCH_3$, $OC_2H_5$) alkoxy ethers (e.g., $[O(CH_2)_nO(CH_2)_mCH_3]$ where n is l to 4, and m is 0 to 4 or $OCH_2CH_2O\ CH_3$ and R, R′, R″ are alkyl or substituted alkyls (e.g., of l to 20 carbon atoms and allowing for ether linkages), aryls or substituted aryl, (e.g., of 6 to 20 carbon atoms) vinyl, acrylate or methacrylate groups. Substituted alkyls include, but are not limited to:

$NH_2-CH_2CH_2NH-CH_2CH_2CH_2-$

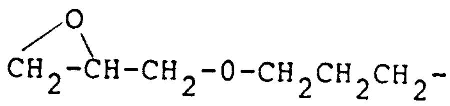

$HS-CH_2CH_2CH_2-$

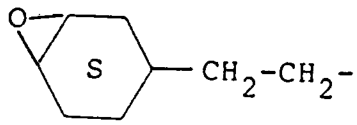

These coupling agents are commercially available.

The particulates or pigments useful in the present invention are preferably finely divided materials having particle sizes of less than 25 microns and preferably less than l0 microns and most preferably less than 2 microns. Suitable materials are non-magentic titanium, iron, aluminum, chromium, copper and cobalt oxides, water insoluble materials such as barium sulfate, a variety of silicates, silica, talcs, carbon black, micas and treated micas, phthalocyanine complexes, and particularly essentially oil and water insoluble cosmetic colorants.

The hydrophobic inner phase for the capsule may be any in situ aminoplast encapsulatable composition as discussed in U.S. Patent 3,5l6,94l. The material or the dispersion produced by incorporation of the particulate may be fluid, semi-solid (e.g., gel), waxy or low melting (less than l00°C) solid carrier phase. Typical materials may be fragrance oils, mineral oils, emollients such as isopropyl myristate, plasticizers such as the phthalate esters, waxes such as found in lipstick, etc.

When the microcapsule is prepared by interfacial polycondensation, the capsule skin may be composed of any condensation polymer or addition polymer, e.g., polyamide, polyurethane, polysulfonamide, polyurea, polyester, polycarbonate, etc. Polyamides prepared by interfacial polycondensation of an amine with an acid chloride or polymers formed by reaction of isocyanate prepolymer with polyamines are preferred. Microcapsules formed by coacervation processes are also useful in forming microcapsule shells according to the present invention. Coacervation is the well known process of forming higher molecular weight gelatin polymers as taught in U.S. Patents 2,800,458 and 2,800,457.

The compositions of the present invention are prepared by making the microcapsules and the cosmetic compositions separately and then mixing the two under conditions which will not rupture a significant (e.g., greater than 5% or l0%) portion of the capsules. Preferably, fewer than l% of the capsules will be broken during preparation of the final composition. In the case of wax or liquid base compositions, intermediate temperatures can be used to soften the compositions prior to mixing with the microcapsules. In powder compositions, the microcapsules and powder can be gently sifted together. In cakes, the pigment and microcapsules may be mixed when dry and then the binder composition added to the mixture. The percentage of capsules and pigments in the composition can vary widely depending on the intensity of the desired effect, the optical intensity of the various pigments, and other aesthetic objectives. Generally, the microcapsules should comprise from l0-90% dry weight of the composition, preferably between 25 and 75% dry weight of the composition. Below are given some particularly preferred ranges in weight percent of useful components and specifically desired compositions for various cosmetic uses.

<u>Face Powder (loose)</u>: primary pigment 20-75%, secondary pigment 25-80%

| | |
|---|---|
| Brilliant lake red (Color Index l5800) | 45% |
| fused silica (flow agent) | 1% |
| Capsule (l0-30 microns, 85% payload of ferric ferrocyanide, Color Index 77510 and diethyl phthalate | 54% |

Face Powder (compact): Binder (2-25%), primary pigment 20-75%, Microcapsules 78-10%

| | |
|---|---|
| Carmine (Color Index 75470) | 75% |
| lipid (as binder) | 5% |
| encapsulated titanated mica (glitter) in diethylphthalate | 20% |

Cream Rouge: 35-65% Cream Base (e.g., water, polyol, lipid, sufactant-emulsifier, preservative and perfume), l5-50% primary pigment, l5-50% microcapsules

| | |
|---|---|
| Ultramarine (Color Index 77007) | 35% |
| Cream base | 45% |
| microcapsules (sunset yellow aluminum lake Color Index l5985) in diethylphthalate | 20% |

Eye Shadow (Compact Powder): 30-70% talc (as powder base), 2-12% metal carboxylate (e.g., zinc stearate) as adherent, 0-3% perfume carrier, 2-10% wax or lipid binder, 0-12% titanium dioxide for covering power, 4-30% primary pigment, and 3-30% microcapsules

| | |
|---|---|
| talc | 38% |
| zinc stearate | 6% |
| lipid binder | 5% |
| titanium dioxide | 5% |
| primary pigment (ultramarine (Color Index 77007) | 30% |
| microcapsules (litholrubin B, barium lake, Color Index 15850) | 16% |

Lipstick: 30-70% lipid-wax mixture (e.g., carnauba wax, beeswax), 15-40% lipid solvent (e.g., ricinus oil, 3-15% primary pigment, 3-15% microcapsules

The capsules used in these constructions and generally in the practice of the present invention have average diameters between 4 and 100 microns. Preferably the average diameters are between 10 and 80 microns. The capsules preferably constitute from 20 to 60% by volume or weight of the composition layer, most preferably between 25 and 50% by weight or volume of said composition layer.

## Claims

**1.** A color adjustable cosmetic composition comprising a first colorant, a binder, and frangible microcapsules comprising a shell, an encapsulated liquid and a second colorant which differs in color from said first colorant, wherein the cosmetic composition undergoes a visible color change only upon rupture of the microcapsules, said microcapsules being rupturable by rubbing of the composition after it has been applied to skin.

**2.** A composition according to claim 1 wherein said second colorant is predominantly encapsulated by said shell.

**3.** A composition according to either of claims 1 and 2 being a compact face powder comprising 2-25% by weight binder, 20-75% by weight first colorant, and 10-78% by weight of microcapsules.

**4.** A composition according to either of claims 1 and 2 being a cream rouge comprising 35-65% cream base, 15-50% first colorant, and 15-50% microcapsules.

**5.** A composition according to either of claims 1 and 2 being a compact eye shadow comprising 30-70% talc, 2-12% metal carboxylate, 2-10% of a binder selected from waxes and lipids, 4-30% first colorant,

and 3-30% microcapsules.

6. A composition according to claim 5 further comprising up to 12% by weight of a white pigment.

7. A composition according to claim 6 wherein said white pigment is titanium dioxide.

8. A composition according to any one of claims 1 to 7 further comprising a perfume carrier.

9. A composition according to either of claims 1 and 2 being a lipstick comprising 30-70% by weight of a binder selected from lipids and waxes, 15-40% by weight of a solvent for said binder, 3-15% by weight of first colorant, and 3-15% by weight of microcapsules.

10. A process for coloring the skin of a human being comprising applying the composition of any one of claims 1 to 9 to a surface and subsequently applying pressure to said composition to rupture microcapsules and change the apparent color of the composition, said composition being a cosmetic selected from the group consisting of eye shadow, lipstick, pancake, face powder, lip balm, cream rouge, mask powder, and suntan lotion.

**Revendications**

1. Composition cosmétique ajustable colorante comprenant un premier colorant, un liant et des microcapsules frangibles comprenant une coque, un liquide encapsulé et un second colorant qui diffère en couleur dudit premier colorant, où la composition cosmétique ne subit une modification de couleur visible que lors de la rupture des microcapsules, lesdites microcapsules pouvant être brisées par frottement de la composition après qu'elle ait été appliquée à la peau.

2. Composition selon la revendication 1, dans laquelle ledit second colorant est de façon prédominante encapsulé par ladite coque.

3. Composition selon l'une ou l'autre des revendications 1 et 2, qui est une poudre pour visage compacte comprenant 2-25% en poids de liant, 20-75% en poids du premier colorant et 10-78% en poids de microcapsules.

4. Composition selon l'une quelconque des revendications 1 et 2, qui est un rouge de crème comprenant 35-65% de base de crème, 15-50% d'un premier colorant et 15-50% de microcapsules.

5. Composition selon l'une quelconque des revendications 1 et 2, qui est une ombre à paupières compacte comprenant 30-70% de talc, 2-12% de carboxylate métallique, 2-10% d'un liant choisi parmi les cires et les lipides, 4-30% d'un premier colorant et 3-30% de microcapsules.

6. Composition selon la revendication 5, comprenant en outre jusqu'à 12% d'un pigment blanc.

7. Composition selon la revendication 6, dans laquelle ledit pigment blanc est le dioxyde de titane.

8. Composition selon l'une quelconque des revendications 1 à 7, comprenant en outre un support de parfum.

9. Composition selon l'une quelconque des revendications 1 et 2, qui est un rouge à lèvres comprenant 30-70% en poids d'un liant choisi parmi les lipides et les cires, 15-40% en poids d'un solvant pour ledit liant, 3-15% en poids du premier colorant et 3-15% en poids de microcapsules.

10. Procédé de coloration de la peau d'un être humain, dans lequel on applique la composition de l'une quelconque des revendications 1 à 9 à une surface, puis on applique une pression à ladite composition afin de briser les microcapsules et de modifier la couleur apparente de la composition, ladite composition étant un cosmétique choisi dans le groupe constitué par l'ombre à paupières, le rouge à lèvres, le fond de teint, la poudre pour visage, le baume à lèvres, le rouge de crème, la poudre de masque, et la lotion bronzante.

**Patentansprüche**

1. Farbeinstellbare kosmetische Zusammensetzung mit einem ersten Färbemittel, einem Bindemittel und zerbrechlichen Mikrokapseln, die eine Schale, eine eingekapselte Flüssigkeit und ein zweites Färbemittel besitzen, dessen Farbe sich von der dem ersten Färbemittels unterscheidet, dadurch gekennzeichnet, daß die kosmetische Zusammensetzung eine sichtbare Farbveränderung nur bei einem Bruch der Mikrokapseln erfährt, die durch ein Verreiben der auf die Haut aufgetragenen Zusammensetzung zerbrechbar sind.

2. Zusammensetzung nach Anspruch 1, dadurch gekennzeichnet, daß das zweite Färbemittel überwiegend in der Schale eingekapselt ist.

3. Zusammensetzung nach Anspruch 1 oder 2 in Form eines kompakten Gesichtspuders mit 2 bis 25 Gew.-% Bindemittel, 20 bis 75 Gew.-% erstes Färbemittel und 10 bis 78 Gew.-% Mikrokapseln.

4. Zusammensetzung nach Anspruch 1 oder 2 in Form eines Cremerouges mit 35 bis 65% Cremebasis, 15 bis 50% erstes Färbemittel und 15 bis 50% Mikrokapseln.

5. Zusammensetzung nach Anspruch 1 oder 2, in Form von Lidschatten-Kompakts mit 30 bis 70% Talkum, 2 bis 12% Metallcarboxylat, 2 bis 10% aus Wachsen und Lipiden ausgewähltes Bindemittel, 4 bis 30% erstes Färbemittel und 3 bis 30% Mikrokapseln.

6. Zusammensetzung nach Anspruch 5, die ferner bis zu 12 Gew.-% weißes Pigment enthält.

7. Zusammensetzung nach Anspruch 6, dadurch gekennzeichnet, daß das weiße Pigment Titandioxid ist.

8. Zusammensetzung nach einem der Ansprüche 1 bis 7, die ferner einen Duftstoffträger enthält.

9. Zusammensetzung nach Anspruch 1 oder 2 in Form eines Lippenstiftes mit 30 bis 70 Gew.-% aus Lipiden und Wachsen ausgewähltes Bindemittel, 15 bis 40 Gew.-% Lösungsmittel für das Bindemittel, 3 bis 15 Gew.-% erstes Färbemittel und 3 bis 15 Gew.-% Mikrokapseln.

10. Verfahren zum Färben der Haut eines Menschen, in dem die Zusammensetzung nach einem der Ansprüche 1 bis 9 auf eine Fläche aufgetragen und danach auf die Zusammensetzung ein Druck ausgeübt wird, um Mikrokapseln zu zerbrechen und die scheinbare Farbe der Zusammensetzung zu verändern, wobei die Zusammensetzung ein Kosmetikum ist, das aus der Gruppe ausgewählt ist, die aus Lidschatten, Lippenstift, festem Puder-Make-up, Gesichtspuder, Lippenbalsam, Cremerouge, Maskenpuder und Sonnenbräunungsflüssigkeit besteht.